# EUROPEAN PATENT SPECIFICATION

(11) **EP 0 807 618 B1**
(45) Date of publication and mention of the grant of the patent: **03.09.2003**
(21) Application number: 97303410.1
(22) Date of filing: 19.05.1997
(51) Int. Cl.: C07C 51/265, C07C 45/36, C07C 29/50, C07C 51/43, C07C 45/82, C07C 29/80

(54) **A method for preparing aromatic carboxylic acids, aromatic aldehydes and aromatic alcohols**
Verfahren zur Herstellung von aromatischen Carbonsäuren, aromatischen Aldehyden und aromatischen Alkoholen
Procédé pour la préparation d'acides carboxyliques aromatiques, d'aldéhydes aromatiques et d'alcools aromatiques

(30) Priority: 17.05.1996 JP 12314796; 17.05.1996 JP 12314896; 17.05.1996 JP 12314996
(43) Date of publication of application: 19.11.1997
(73) Proprietor: TORAY INDUSTRIES, INC., Tokyo 103-8666 (JP)
(72) Inventor: Matsuoka, Shotaro, Mizuho-ku, Nagoya-shi, Aichi, 467 (JP); Suematsu, Masaaki, Nissin-shi, Aichi, 470-01 (JP); Kitamura, Akira, Midori-ku, Nagoya-shi, Aichi, 458 (JP)
(74) Representative: Coleiro, Raymond

(56) References cited:
- EP-A- 0 071 166
- FR-A- 2 379 500
- GB-A- 970 492
- CHEMICAL ABSTRACTS, vol. 101, no. 9, 27 August 1984 Columbus, Ohio, US; abstract no. 72434w, page 616; XP002094556 & JP 59 053442 A (NIPPON KAYAKU CO., LTD.)
- CHEMICAL ABSTRACTS, vol. 108, no. 3, 18 January 1988 Columbus, Ohio, US; abstract no. 21398z, CVENGROSOVA Z ET AL: "Study of p-xylene reaction with cobalt-bromine-pyridine catalyst. Part II. Reaction in aqueous system" page 553; XP002094557 -& DATABASE CHEMICAL ABSTRACTS ACS XP002094560 & J. MOL. CATAL., vol. 40, no. 2, 1987, pages 235-42,
- CHEMICAL ABSTRACTS, vol. 103, no. 3, 22 July 1985 Columbus, Ohio, US; abstract no. 22286v, page 547; XP002094558 & JP 60 019736 A (TORAY INDUSTRIES, INC.)
- CHEMICAL ABSTRACTS, vol. 95, no. 19, 9 November 1981 Columbus, Ohio, US; abstract no. 168804a, page 701; XP002094559 & JP 56 055341 A (NIHON JORYU KOGYO CO., LTD.)

## Description

The present invention relates to a method for preparing aromatic carboxylic acids, aromatic aldehydes, and aromatic alcohols, wherein the aromatic carboxylic acids, aromatic aldehydes, and aromatic alcohols are compounds represented by following formulae ( II ), (III), and (IV); 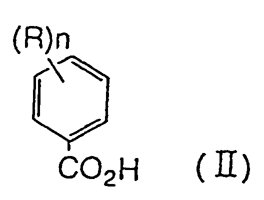 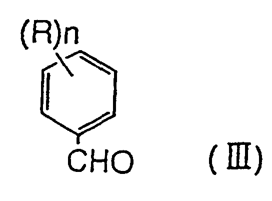 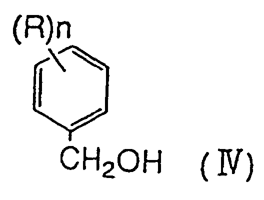 wherein n represents any of integer 1 to 5; and substituent R represents a C₁₋₁₈ alkyl group, a C₆₋₁₈ aryl group, a C₆₋₁₈ aralkyl group or a haloalkyl group.

The aromatic carboxylic acids, aromatic aldehydes, and aromatic alcohols are all regarded as industrially important raw materials for pharmaceutical and agricultural products, and polymer products.

Reactions for producing the aromatic carboxylic acids from aromatic compounds having a methyl group, such as chlorotoluene, nitrotoluene, and the like, by oxidation, such as air oxidation, are well known. Furthermore, it is also well known, that, in such reactions, industrially important byproducts, such as aromatic aldehyde, aromatic alcohol, etc., are produced.

GB 1561464 proposes a method for oxidation of p-toluene derivatives especially for the preparation of p-substituted benzaldehydes. The oxidation takes place in the presence of a cobalt compound, a bromine compound and a carboxylic acid.

In the oxidising reaction, in an attempt to produce effectively the desired aromatic carboxylic acid by suppressing the formation of various intermediate oxidizing products, several approaches have been tried so far. Examples are a method for producing p-toluic acid by oxidation with a gas containing oxygen, in the presence of a specific shaped catalyst of cobalt oxide (GB-B-1005315) and a method for producing benzoic acid derivatives by liquid phase oxidation of toluene derivatives with a gas containing oxygen in the presence of a phase transfer catalyst composed of a quaternary onium salt and a transition metal salt, together with a small amount of polar solvent, which is capable of dissolving the catalyst (JP-A-01-2287054).

EP-A-0071166 also concerns the oxidation of methyl substituted aromatic compounds using a cobalt catalyst to prepare aromatic aldehydes. A reaction solution containing aromatic acid, aromatic aldehyde and aromatic alcohol is subjected to subsequent separation processes.

It is well known that an aromatic carboxylic acid may be obtained by distilling, sedimenting with acid, or crystallizing reacting solution, or that an aromatic aldehyde and an aromatic alcohol may also be obtained by directly distilling the reaction solution, or by extracting the desired component from the reaction solution using an extracting solution, followed by rectification thereof (JP-B-48-023430, JP-A-53-082734, JP-A-54-079244, JP-B-62-002576, JP-B-62-030974, JP-B-62-030975, JP-A-02-073038, JP-B-04-003370, JP-B-07-116096 and WO-A-95/20560).

Of the aromatic carboxylic acids, p-toluic acid may be produced by oxidizing p-xylene. As the byproduct of the reaction, p-methylbenzyl alcohol, p-tolualdehyde, p-hydroxymethylbenzoic acid, 4-carboxybenzaldehyde, terephthalic acid and the like, may be formed.

For purifying p-toluic acid thus produced, physical processes, such as distillation, extraction and recrystallization, which are conventionally used in purification procedures in general, have not been applied to p-toluic acid, due to the sublimable property of p-toluic acid, or due to its ready solubility in various solvents.

As a purifying process for p-toluic acid, a process is known, which comprises treating at a high temperature crude p-toluic acid solution in an organic solvent, which is capable of selectively reacting in forming a salt of the byproducts, and removing the salt, thus produced, in water extraction, and cooling the same (JP-A-54-079244). Subsequently, washing the crystals with n-hexane is carried out to further improve the purity of the p-toluic acid.

However, since an oxidizing reaction according to the conventional method is exclusively aimed at effectively producing aromatic carboxylic acids from aromatic hydrocarbons, regardless of the fact that some of the intermediates in the oxidation process, for example, aromatic aldehyde and aromatic alcohol, are useful as a raw material for pharmaceutical or agricultural products, the intermediates are disposed of as impurities, and are not effectively utilized.

On the other hand, effective isolation of particular compounds from reacting solutions containing these compounds, cannot be said to have been sufficiently well conducted to date. That is, since when an aromatic carboxylic acid is to be produced, all compounds other than the aromatic carboxylic compound, which include aromatic aldehydes and aromatic alcohols, are considered as impurities, only a purification of the aromatic carboxylic acid is to be carried out.

However, the aromatic aldehyde or aromatic alcohol is reacted and decomposed, and is liable adversely to affect the purification of the acid. A similar phenomenon occurs in the process for obtaining an aromatic aldehyde and an aromatic alcohol. As a specific example, it is well known that if, generally, an aldehyde and alcohol are heated under acidic conditions, an acetal is formed with 1 mole of aldehyde and 2 mole of alcohol. This reaction is thus liable to occur when purifying aromatic aldehyde and aromatic alcohol under distillation. By forming acetal in the distillation, the yield of the aromatic aldehyde and aromatic alcohol may be reduced, resulting in both lower yield and lower purity. Thus, it is difficult effectively to obtain all three types of compound according to a combination of simple unit operation.

In addition to the above, among the aromatic carboxylic acids, p-toluic acid suffers a significantly large loss of the product in the washing operation, due to solubility thereof. In this connection, although p-methylbenzyl alcohol and p-tolualdehyde, which are byproducts, may form p-toluic acid by subsequent oxidation, these byproducts have never been recovered, nor utilized, so far.

An object of the present invention is to provide a method for efficiently preparing an aromatic carboxylic acid, simultaneously with an aromatic aldehyde and an aromatic alcohol.

According to one aspect, the invention provides a method for preparing at least one aromatic carboxylic acid, aromatic aldehyde and aromatic alcohol, by oxidizing an aromatic compound represented by formula (I), which is as follows: 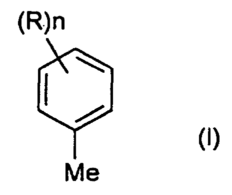 wherein n represents any integer of 1 to 5; and each substituent R is C₁₋₁₈ alkyl, C₆₋₁₈ aryl, C₆₋₁₈ aralkyl or haloalkyl. The oxidation is carried out with a gas containing molecular oxygen, in the presence of catalyst composed of transition metal compound, a tertiary amine and a bromide compound wherein the transition metal compound is present in an amount of 0.2 wt% or less, calculated as an amount of the transition metal, with respect to the weight of the raw material and the weight ratio of the transition metal compound:tertiary amine is 1:0.1 to 1:100.

The oxidation may be carried out in the liquid phase.

According to an embodiment, the present invention provides a method for efficiently obtaining an aromatic aldehyde by an acid depositing, or crystal precipitating treatment upon reaction solution, which is obtained by the above method of oxidizing the compound represented by formula (I), and simultaneously obtaining aromatic aldehyde and aromatic alcohol from the filtrate by a treatment, such as distillation.

According to further embodiments, the present invention provides a method for attaining a preferable result of the above oxidation method by removal of acid, which would otherwise have acted as a catalyst in an acetal forming reaction, by adding base into a mixture solution containing aromatic aldehyde and aromatic alcohol to be distilled, in order not to suppress production of aromatic aldehyde and aromatic alcohol in distillation step.

According to yet further embodiments, the present invention provides a method for efficiently preparing aromatic carboxylic acid of high purity, which comprises purifying crude aromatic hydrocarbons, obtained by the above oxidizing method, by washing with raw aromatic hydrocarbons, and simultaneously using in a recycled manner, as a raw material, from which solution and crude aromatic carboxylic acid are removed by a washing step.

Embodiments of the present invention are now explained in more detail below.

In the oxidizing reaction of the present invention, at least one of the aromatic hydrocarbons represented by the following formula (I) is used as a raw material. 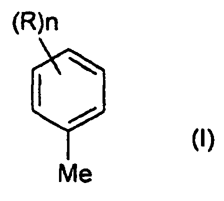 **(wherein n, R**^{**1**}**, R**^{**2**} **and R**^{**3**} **are as defined above)**

Furthermore, raw materials comprising at least one of aromatic hydrocarbons, or oxidation products thereof, wherein substituent R in the formula (I) represents any of an alkyl group, an aryl group, or an aralkyl group, are preferably used.

For example, xylene, methylbenzaldehyde, and methylbenzyl alcohol are preferably used as a raw material.

The reaction is characterized by oxidation, which may be liquid phase oxidation, with a gas containing molecular oxygen, in the presence of catalyst comprising transition metal compound, tertiary amine, and bromide compound, and is to produce aromatic carboxylic acid, aromatic aldehyde, and aromatic alcohol, simultaneously.

The amount of the transition metal compound to be used is less than 0.2 wt %, calculated as transition metal, with respect to the raw material. If the amount is above this, the load-in operation cost and cost for separating the transition metal compound from the raw material may be increased.

'The transition metal present in the transition metal compound is preferably selected from 5 to 10 groups in the long-form Periodic Table, and several elements may be used in combination to further improve catalytic activity.

At least one of manganese, tungsten, molybdenum, chromium, vanadium, cobalt, and cerium are preferable, and cobalt is the most preferable.

No specific problem may arise from using any of the transition metal compounds, if the compound contains the transition metals discussed above.

However, having regard to solubility in the reaction system, or availability in the commercial market, chlorides, bromides, acetates, and sulfates thereof are preferred.

Examples of the preferred transition metal compounds include cobalt chloride, cobalt bromide and cobalt actetate.

The weight ratio of the transition metal compound to tertiary amine is within a range of 1:0.1 to 1:100, 'preferably 1:1 to 1:30, still more preferably 1:5 to 1:20. Since even if an excess amount of the tertiary amine may be used, no remarkable advantage may be attained, so the use thereof in an excess amount should be avoided.

The kind of tertiary amine to be used has no specific limit; however, the use of an aromatic amine such as pyridine, picoline, lutidine and xynoline, or an aliphatic amine, such as tributylamine and triethylamine, are preferred.

The weight ratio of the transition metal compound to the bromide compound is preferably within a range of 1:0.1 to 1:100, more preferably 1:1 to 1:30, still more preferably 1:5 to 1:20. The use of the bromide compound in a large amount is not preferable because it may cause corrosion of apparatus.

Examples of the bromide compound include inorganic bromide compounds, such as hydrogen bromide, alkali metal bromide and ammonium'bromide, and organic bromide compounds, such as tetrabromoethane, bromoacetate and bromobenzyl, as available.

According to the present invention, a small amount of water may be added into a charged mixture, containing raw material and catalyst. The amount thereof is 0.01 to 20 wt % with respect to the amount of the raw material, more preferably 1.0 to 10 wt%. By the addition of the water, selectivity of the aromatic alcohol is improved.

The oxidation reaction of the present invention may be carried out at a temperature of 100 to 250°C, preferably 140 to 200°C. With higher temperatures a side reaction may be caused, whereas with lower temperatures the reaction rate may be remarkably lowered.

As for the gas containing molecular oxygen, although pure oxygen or industrial exhaust gas may be used, in industrial use common air or a mixture of air and industrial exhaust gas are suitably available.

The reaction pressure is preferably within a range of 0.1 to 6MPa, preferably 0.5 to 2MPa, and the oxygen content in the waste gas from the reactor is preferably controlled so as to be below explosion limit in concentration.

The reaction may be carried out in the presence of a solvent, wherein the solvent is preferably inactive under the oxidizing reaction.

A reactor to be used in a method of the present invention is preferably of a forced stirring type, rather than the simple bubbling tower type. That is, in order to accelerate dissolution of the gas containing molecular oxygen into the reaction mixture, and smoothly contact reactants with each other, in the reactor, it is preferable to use a reactor which is provided with many gas blowing nozzles at its lower part and is capable of operating in a forced stirring with a revolving stirring vane, or circulating pump provided at the outside thereof.

At the upper part of the reactor, a reflux condenser is provided so that the waste gas may be exhausted through the reflux condenser, and the solvent, raw material, etc. which are accompanied with the waste gas are condensed and recycled to the reactor.

As one of the most typical examples of the procedure for the reaction steps, a process, which comprises heating the aromatic hydrocarbons, i.e. raw material, in the presence of the catalyst, comprising transition metal compound, tertiary amine, and bromide compound, and introducing gas containing molecular oxygen gas, is now shown. When the oxidation reaction is carried out as far as consuming the raw material to 10 to 70% (conversion of 10 to 70%), which generally takes about 0.5 to 10 hours, the aromatic carboxylic acid, aromatic aldehyde, which is an intermediate, and aromatic alcohol are formed in selectivity of 20 to 70%, 10 to 40%, and 5 to 30%, respectively, and reaction product solution, containing small amount of the byproducts, etc., may be obtained.

In separation and purification steps for the aromatic carboxylic acid, aromatic aldehyde, and aromatic alcohol, according to the present invention, a reaction solution obtained by oxidizing the aromatic compounds is used.

As for the aromatic compound used in the present invention as a raw material, any of the compounds represented by the formula (I) may be used. Specific examples thereof include xylene, ethyl toluene, cumene, butyl toluene, trimethyl benzene and tetramethyl benzene, and preferably xylene.

To obtain oxidizing reaction solution used in the present invention, other than the liquid phase reaction using a gas containing molecular oxygen, in the presence of the transition metal, such as cobalt, manganese, etc., a gas phase oxidation, or a liquid phase or gas phase oxidation using oxidizing agents, such as permanganic acid, chromic acid, hydrogen peroxide, nitric acid, etc., may be used.

Among these, the liquid phase oxidation reaction using a gas containing molecular oxygen is the most preferred.

The reaction solution, obtained by the above process, is separated into the aromatic carboxylic acid and residue containing aromatic aldehyde, and aromatic alcohol, by cooling or crystallizing, after condensing thereof, on demand.

At the same time, the aromatic carboxylic acid further purified, may be obtained by recrystallization of the crude aromatic carboxylic acid.

Alternatively, depending on the process, the aromatic carboxylic acid further purified, may be obtained by treating the reaction solution with a base to separate the salts obtained, i.e. compounds which are to be impurities of the aromatic carboxylic acid, aromatic aldehyde, and aromatic alcohol, followed by cooling and crystallization.

Examples of the base, which may be used for treating the reaction solution, include an aqueous solution of sodium hydroxide, potassium hydroxide, calcium hydroxide, potassium carbonate, calcium carbonate and sodium hydrogencarbonate, and an aqueous solution of organic basic compounds, such as pyridine, picoline, triethylamine, tripropylamine and tributylamine. These bases may be used either alone or in combination. The treating temperature has no specific limit; however, having regard to operability or probability in causing side reaction, the temperature is preferably as low as possible, which is 60 to 120°C. Furthermore, the reaction time has also no specific limit, since the reaction is for simple neutralization; however, having regard to the probability of causing the side reaction, it is not necessary to carry out the reaction for a long time, and 5 to 60 minutes may be sufficient. In this connection, the amount of the base to be used is from 1.0 to 10.0 times the molar amount of the impurity, preferably 3.0 to 7.0 times. The amount of water, which is required for dissolving the base, is also not specifically limited; however, having regard to loss of the effective component, an amount of 0.05 to 0.3 wt % of the reaction solution may be sufficient for removing the impurity. From the stand point for removing the impurity, a repeated treatment of the same operation is acceptable.

Depending on the process, in advance of the cooling and crystallization, an impurity dissolved in a water layer part, wherein the water is derived from a byproduct in the oxidation reaction, is preferably removed previously.
Under the circumstances, after adding water from outside and stirring the mixture, it is further preferable, so as more certainly to remove the impurity, to remove the water phase.. Furthermore, the water phase obtained by the above process may be available for recycling to the original oxidation reaction depending on the process, since the water phase is mainly composed of the catalyst component.

On the other hand, by distilling a filtrate after crystallization, the aromatic aldehyde and aromatic alcohol may be obtained. At the same time, by distilling a solution obtained after removing the acid component, such as aromatic carboxylic acid, etc., contained, the aromatic aldehyde and aromatic alcohol are able to be more efficiently obtained.

As for a technical process for removing the acid component, such as aromatic carboxylic acid, etc., if an acid component shows volatility, and is capable of being removed by a distilling condensation process, almost all of said component may be so removed. On the other hand, if the acid component would be difficult to remove by condensation, the component may be removed by any method of repeatedly conducting condensing and crystallization, neutralizing removal with required amount of the base, absorbing removal with ion-exchange resin, or the like. In other words, depending on the nature of the acid component, various methods may be considered; however, a preferable method, easily operable, and capable of removing a small amount of the acid component, is a neutralizing removal with a required amount of the base, regardless of whether the acid component may be volatile, or not.

The base, which may be used in the removal process, includes an aqueous solution of hydroxide, such as sodium hydroxide, potassium hydroxide, calcium hydroxide, sodium carbonate, potassium carbonate and calcium carbonate, and an aqueous organic basic compound, such as pyridine, picoline, triethylamine, tripropylamine and tributylamine. These bases may be used alone, or in combination. The treating temperature has no specific limit, provided that a residue may be maintained in a liquid state, and the neutralization reaction may proceed; however, having regard to the capability for causing side reaction, the temperature is preferably as low as possible, i.e. within a range of 60 to 120°C. The treating time has also no specific limit because the reaction is simple; however, having regard to the capability for causing side reaction, it is not necessary to carry out the reaction for a long time, and 30 to 120 minutes is sufficient. In this connection, with a molar amount of the base of from 1.0 to 10.0 times the molar amount of the acid component, preferably 1.0 to 3.0 times, the acid component can be removed.

However, according to the above process, although almost all of residual acid components will be removed, it is difficult to remove very small amounts thereof.

According to the present invention, in order to efficiently obtain aromatic aldehyde and aromatic alcohol, since the acid component can function as a catalyst to form an acetal, thereby lowering a purity and yield of the aromatic aldehyde and aromatic alcohol, no more than a small amount of the acid component may be present.

Accordingly, in order to remove small amounts of the acid, which would otherwise become a catalyst for acetal forming reaction, a base is added to a solution containing aromatic aldehyde and aromatic alcohol, which are raw materials, on distilling the solution. The acid component in the distilling raw material is captured by the base to form salt, thereby losing catalytic activity in acetal forming reaction. As for the acid, an organic acid such as a carboxylic acid, etc., which is formed by the oxidizing reaction, and inorganic acid derived from a catalyst component in the oxidation reaction may be adopted.

Examples of the base to be used include aqueous solutions of hydroxides, such as sodium hydroxide, potassium hydroxide, calcium hydroxide, sodium carbonate, potassium carbonate and calcium carbonate and aqueous organic base compounds, such as pyridine, picoline, triethylamine, tripropylamine and tributylamine. These bases may be used alone or in combination. Having regard to easy availability and avoidance of contaminating the final product, sodium hydroxide is preferably used as a base in the form of an aqueous solution.

The amount of the base to be added is 10 wt% or less, preferably 1 wt% or less with respect to a total amount of the aromatic aldehyde and aromatic alcohol. If the base is used in excess, impurity will be increased due to side reaction. Furthermore, the aromatic alcohol is liable to be decomposed by the base. If the base is not used in an amount of 1 or more mole ratio, with respect to acid component in the solution containing the aromatic aldehyde and aromatic alcohol, the suppression effect for forming acetal will be lowered.

Furthermore, the residue obtained by the above distilling operation may be recycled to be used in the original oxidation reaction, depending on the operation. It is also possible to isolate effective aromatic carboxylic acid from the solution containing acid component.

According to the present invention, the aromatic carboxylic acid in high purity is efficiently prepared by washing and purifying the crude aromatic carboxylic acid, which was obtained by oxidizing aromatic hydrocarbons having at least one, methyl group, with the raw aromatic hydrocarbons, and simultaneously by using the solution, which is obtained after washing operation, and from which the crude aromatic carboxylic acid is removed, in recycled manner, as a raw material.

The aromatic hydrocarbon used in the present invention as a raw material are aromatic hydrocarbons having at least one methyl group as previously defined, and the aromatic carboxylic acid produced may be a benzoic acid substituted with an alkyl group. Examples of the raw material preferably include xylene, more preferably p-xylene, and example of the product preferably includes toluic acid, more preferably p-toluic acid.

The compound used for washing p-toluic acid produced may be p-xylene, which is raw material, in an amount chosen so as to be sufficient for washing an impurity out, which is adsorbed over the crystal surface of the p-toluic acid, but so as to not dissolve large amount of the p-toluic acid.

The filtrate after obtaining toluic acid, and the washing solution for toluic acid, may be combined together to be used as a raw material for the subsequent reaction. The residual impurities remaining in the combined solution; show no adverse affect on a reaction to obtain p-toluic acid.

As discussed above, by establishing the method according to the present invention, the aromatic carboxylic acid, aromatic aldehyde, and aromatic alcohol are able to be simultaneously and efficiently produced. Embodiments of the present invention are further explained in more detail, with reference to the following Examples. Unless otherwise specified, a part, ratio or percent is by weight.

### Example 1

In to a 1 1 autoclave made of titanium, having a reflux condenser and a revolving vane stirrer, 500 g of p-xylene (produced by Kishida Kagaku, special grade), 0.23 g of cobalt chloride 6 hydrate (Katayama Kagaku, special grade), 0.54 g of pyridine (Kishida Kagaku, special grade), and 1.18 g of hydrobromic acid were charged, and contacted with air, blown under 1.4 MPa of reaction pressure, at a reaction temperature of 170°C for 6 hours, at such a rate that the oxygen content in the exhaust gas may be 3% or lower, and the flow rate of the exhaust gas may be 0.6 Nl/min.

According to the results of analysis, the conversion of p-xylene in this oxidation reaction was 46.8% by gas chromatography, the selectivity of p-toluic acid by high performance liquid chromatography was 46.1% and the selectivity of p-tolualdehyde and p-methylbenzyl alcohol by gas chromatography was 12.3%, and 8.9% respectively.

### Example 2

The process was repeated in the same manner as that of Example 1, except that 0.13 g of tributylamine (Kishida Kagaku, special grade) was used in place of the pyridine, at a reaction temperature of 155°C.

According to the results of analysis, the conversion of p-xylene in this oxidizing reaction was 54.0% by gas chromatography, the selectivity of p-toluic acid by high performance liquid chromatography was 57.7% and the selectivity of p-tolualdehyde and p-methylbenzyl alcohol by gas chromatography was 10.7%, and 8.3%, respectively.

### Example 3

The process was repeated in the same manner as that of Example 1, except that 0.71 g of sodium bromide (Katayama Kagaku, Special grade) was used in place of the hydrobromic acid.

According to the results of analysis, the conversion of p-xylene in this oxidizing reaction was 48.2% by gas chromatography, the selectivity of p-toluic acid by high performance liquid chromatography was 47.7% and the selectivity of p-tolualdehyde and p-methylbenzyl alcohol by gas chromatography was 11.8%, and 7.9%, respectively.

### Example 4

In the process of the Example 1, 15 g of water (3% with respect to raw p-xylene) was added.

According to the results of analysis, the conversion of p-xylene in this oxidation reaction was 27.3% by gas chromatography, the selectivity of p-toluic acid by high performance liquid chromatography was 39.1% and the selectivity of p-tolualdehyde and p-methylbenzyl alcohol by gas chromatography was 16.5%, and 13.6%, respectively.

### Example 5

To a pressure resistive apparatus of glass, provided with an liquid inlet tube, which is capable of charging alkali solution, etc., a sampling tube, which is capable of withdrawing liquid from lower layer, and a revolving vane stirrer, 524 g of the reaction mixture, which was obtained according to the same condition as of the Example 1, was charged, and the pressure was raised to 0.2 MPa.G with nitrogen gas, followed by raising the temperature to 130°C (pressure was raised to about 0.5 MPa.G). Then, 15 minutes after standing still, 4.0 g of a water layer remaining in the lower layer, was drawn out through the sampling tube. After that, 71.9 g of 4.9% aqueous sodium hydroxide solution (which was 0.14 times with respect to the reaction solution; the molar amount of the sodium hydroxide was 4.8 times with respect to total amount of terephthalate and p-carboxybenzaldehyde) was added, stirred at 110°C, for 45 minutes (the pressure was about 0.3 MPa G), and similarly, after standing still for 15 minutes, 76.4 g of the water was drawn. Furthermore, 74.3 g of ion-exchanged water (0.14 times with respect to the reaction solution) was charged, stirred at 110°C, for 30 minutes (the pressure was about 0.3 MPa G), and after standing still for 15 minutes, 82.6 g of the water layer was drawn. After the processes above, the reaction solution was cooled to about 30°C, filtered with suction as it is, to obtain 69.6 g of cake and 418.6 g of filtrate. As for the cake, 102.2 g of p-xylene (1.5 times with respect to the cake) was added, and after stirring at 40 °C for 30 minutes, a process for filtration under suction and drying was carried out to obtain 52.4 g of p-toluic acid as white crystal (63.4% in isolated yield with respect to the toluic acid in the reacting solution). The p-toluic acid obtained showed 98.8% in purity, and 96.1% in transmission at 460 nm.

On the other hand, the results of analysis according to the high performance liquid chromatography showed compositions, as stated below:

| | |
|---|---|
| p-Xylene | 70.2 % |
| p-Toluic acid | 3.8 % |
| p-Tolualdehyde | 6.6 % |
| p-Methylbenzyl alcohol | 4.8 % |
| Terephthalic acid | < 0.1 % |
| p- Carboxybenzaldehyde | < 0.1 % |
| p-Hydroxymethylbenzoic acid | < 0.1 % |
| p-Methylbenzyl toluate | < 0.1 % |
| Other | 10.8 % |

### Examples 6 and 7

A study of the method of Example 1 was carried out in the same manner, except that the concentration of the sodium hydroxide was varied. The results for the crystallized p-toluic acid obtained are shown in Table 1 below.

**Table 1**

| | Molar Factor* of NaOH | p- Toluic Acid | | |
|---|---|---|---|---|
| | (mol/mol) | Yield (%) | Purity (%) | Transmission at 460nm ( % ) |
| Example 6 | 3.4 | 64.2 | 98.5 | 96.8 |
| Example 7 | 5.7 | 55.3 | 98.9 | 96.6 |

| | | | | |
|---|---|---|---|---|
| Remarks: * Shows molar ratio of NaOH with respect to total amount of terphthalate and p-carboxybenzaldehyde. | | | | |

### Example 8

To 2100 g of the reaction filtrate obtained under the same conditions as in Example 1, 364.0 g of aqueous sodium hydroxide solution (the molar amount of sodium hydroxide was 1.5 times with respect to a total molar amount of the p-toluic acid and p-methylbenzyl toluate) was added, stirred at 90 °C for 4 hours, and after standing still 15 minutes, the solution was separated into 1937.5 g of organic layer and 372.0 g of water layer. To the organic layer, 0.27 g of aqueous 40 % NaOH solution (sodium hydroxide was 0.11 % with respect to total amount of the p-tolualdehyde and p-methylbenzyl alcohol contained in the solution) was added, and distilled to obtain 822 g of p-tolualdehyde, and 84.8 g of p-methylbenzyl alcohol. As the result of the analysis by gas chromatography, purities of the p-tolualdehyde and p-methylbenzyl alcohol were found to be 93.3% and 99.5% respectively.

### Example 9

Xylene was added to the washing solution, which was obtained by reacting under the same conditions as in Example 1, and treating with the alkali solution under the same conditions as in Example 5, and the residue obtained under the same conditions as in Example 8, so that the total amount of the p-xylene was to be 500 g, together with that contained in the washing solution, and reacted under the same conditions as in Example 1. As the result of the analysis, the conversion of p-xylene was 34.8% by gas chromatography, the selectivity of p-toluic acid by high performance liquid chromatography was 45.7% and the selectivity of p-tolualdehyde and p-methylbenzyl alcohol by gas chromatography was 15.2%, and 12.0%, respectively. The filtrate and washing solution showed the same reactivity as in the initial reaction, even on recycle use.

## Claims

1. A method for preparing at least one aromatic carboxylic acid, aromatic aldehyde and aromatic alcohol, by oxidizing at least one aromatic compound represented by the formula (I) as follows: 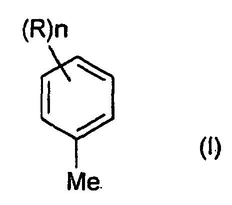 wherein n is an integer of 1 to 5 and each R, independently, is C₁₋₁₈ alkyl, C₆₋₁₈ aryl, C₆₋₁₈ aralkyl or haloalkyl,
the method comprising oxidizing at least one aromatic compound of formula (I) as a raw material with a molecular oxygen containing gas, in the presence of a catalyst comprising a transition metal compound, a tertiary amine and a bromide compound, wherein the transition metal compound is present in an amount of 0.2 wt% or less, calculated as an amount of the transition metal with respect to the weight of the raw material, and the weight ratio of the transition metal compound:tertiary amine is within a range of 1:0.1 to 1:100.

2. A method according to claim 1 wherein, in the formula (I), each R, independently, is C₁₋₁₈ alkyl or C₆₋₁₈ aralkyl.

3. A method according to claim 1 or claim 2, wherein the raw material is at least one compound selected from xylene, tolualdehyde and methylbenzyl alcohol.

4. A method according to any preceding claim, wherein the transition metal is selected from the group of 5 to 10 in the long term Periodic Table.

5. A method according to claim 4, wherein the transition metal is selected from manganese, tungsten, molybdenum, chromium, vanadium, cobalt and cerium.

6. A method according to any preceding claim, wherein the transition metal compound is selected from a chloride, bromide, acetate and sulfate.

7. A method according to claim 6, wherein the transition metal compound is selected from cobalt chloride, cobalt bromide and cobalt acetate.

8. A method according to any preceding claim, wherein the weight ratio of the transition metal compound : bromide compound is within a range of 1 : 0.1 to 1 : 100.

9. A method according to any preceding claim, wherein water is added into a charged reacting solution.

10. A method according to claim 9, wherein the water is present in an amount of 0.01 to 20 wt% with respect to the weight of the raw material.

11. A method according to any preceding claim, wherein the oxidation reaction is carried out at a temperature of 100 to 250°C.

12. A method according to any preceding claim, wherein the oxidation reaction is carried out under pressure of 0.1 to 6 MPa.

13. A method according to any preceding claim, which comprises the additional step of separating an aromatic compound from reaction solution, which aromatic compound is at least one of the said aromatic acid, aromatic aldehyde and aromatic alcohol.

14. A method according to claim 13, wherein the aromatic carboxylic acid is separated by crystallization.

15. A method according to claim 13, wherein the aromatic carboxylic acid is separated by acid precipitation.

16. A method according to claim 13, wherein the aromatic aldehyde and aromatic alcohol are separated from the reaction solution by distillation.

17. A method according to claim 13, wherein the aromatic compound is xylene.

18. A method according to any one of claims 13 to 15, wherein, in advance of separating the aromatic carboxylic acid, an aqueous layer of the reaction solution is separated and removed.

19. A method according to claim 18, wherein the aqueous layer is recycled and used in the oxidizing process.

20. A method according to claim 13, wherein, in advance of cooling and crystal precipitating the reaction solution, the solution is contacted with a base, followed by separating a salt formed.

21. A method according to claim 20, wherein the reaction solution, contacted with the base, is further contacted with water.

22. A method according to claim 16, wherein on distilling the reaction solution, from which the aromatic carboxylic acid is removed, the distillation is carried out after removing acid components.

23. A method according to claim 16, wherein the residue obtained after distillation of the reaction solution is recycled for using in the.oxidizing reaction.

24. A method according to any one of claims 16, 22 and 23, which comprises the further step of distilling the mixture comprising the aromatic aldehyde and the aromatic alcohol additionally containing an aromatic acid in the presence of a base.

25. A method according to claim 24, wherein the aromatic aldehyde is tolualdehyde.

26. A method according to claim 24, wherein the aromatic alcohol is methylbenzyl alcohol.

27. A method according to any one of claims 24 to 26, wherein the base is added in an amount of 10 wt% or less with respect to the total amount of the aromatic aldehyde and aromatic alcohol.

28. A method according to any one of claims 1 to 15, which comprises the additional step of purifying a crude aromatic carboxylic acid obtained by the said method and simultaneously recycling the washing solution for use as a raw material.

29. A method according to claim 28, wherein the aromatic carboxylic acid obtained is an alkyl substituted benzoic acid.

30. A method according to claim 29, wherein the raw aromatic hydrocarbon is xylene, and the aromatic carboxylic acid is toluic acid.

31. A method according to claim 30, wherein the raw aromatic hydrocarbon is p-xylene, and the aromatic carboxylic acid is p-toluic acid.

## Patentansprüche

1. Verfahren zur Herstellung zumindest einer aromatischen Carbonsäure, eines aromatischen Aldehyds und aromatischen Alkohols durch Oxidieren zumindest einer durch die folgende Formel (I) dargestellten aromatischen Verbindung : 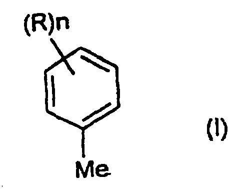 worin n eine ganze Zahl von 1 bis 5 ist und die R jeweils unabhängig voneinander ein C₁₋₁₈-Alkyl, C₆₋₁₈-Aryl, C₆₋₁₈-Aralkyl oder Halogenalkyl sind,
wobei das Verfahren die Oxidation zumindest einer aromatischen Verbindung der Formel (I) als Rohmaterial mit einem molekularen Sauerstoff enthaltenden Gas in Gegenwart eines Katalysators umfasst, der eine Übergangsmetallverbindung, ein tertiäres Amin und eine Bromidverbindung umfasst, worin die Übergangsmetallverbindung in einer Menge von 0,2 Gew.-% oder weniger, berechnet als Menge des Übergangsmetalls bezogen auf das Gewicht des Rohmaterials, enthalten ist und das Gewichtsverhältnis zwischen der Übergangsmetallverbindung und dem tertiären Amin im Bereich von 1:0,1 bis 1:100 liegt.

2. Verfahren nach Anspruch 1, worin die R in Formel (I) jeweils unabhängig voneinander ein C₁₋₁₈-Alkyl oder C₆₋₁₈-Aralkyl sind.

3. Verfahren nach Anspruch 1 oder 2, worin das Rohmaterial zumindest eine aus Xylol, Tolualdehyd und Methylbenzylalkohol ausgewählte Verbindung ist.

4. Verfahren nach einem der vorangegangenen Ansprüche, worin das Übergangsmetall aus den Gruppen 5 bis 10 des Langperiodensystems ausgewählt ist.

5. Verfahren nach Anspruch 4, worin das Übergangsmetall aus Mangan, Wolfram, Molybdän, Chrom, Vanadium, Cobalt und Cer ausgewählt ist.

6. Verfahren nach einem der vorhergehenden Ansprüche, worin die Übergangsmetallverbindung aus einem Chlorid, Bromid, Acetat und Sulfat ausgewählt ist.

7. Verfahren nach Anspruch 6, worin die Übergangsmetallverbindung aus Cobaltchlorid, Cobaltbromid und Cobaltacetat ausgewählt ist.

8. Verfahren nach einem der vorhergehenden Ansprüche, worin das Gewichtsverhältnis zwischen der Übergangsmetallverbindung und der Bromidverbindung im Bereich von 1:0,1 bis 1:100 liegt.

9. Verfahren nach einem der vorhergehenden Ansprüche, worin Wasser zu einer geladenen Reaktionslösung zugesetzt wird.

10. Verfahren nach Anspruch 9, worin das Wasser in einer Menge von 0,01 bis 20 Gew.-%, bezogen auf das Gewicht des Rohmaterials, enthalten ist.

11. Verfahren nach einem der vorhergehenden Ansprüche, worin die Oxidationsreaktion bei einer Temperatur von 100 bis 250 °C durchgeführt wird.

12. Verfahren nach einem der vorhergehenden Ansprüche, worin die Oxidationsreaktion bei einem Druck von 0,1 bis 6 MPa durchgeführt wird.

13. Verfahren nach einem der vorhergehenden Ansprüche, das den zusätzlichen Schritt der Abtrennung einer aromatischen Verbindung von der Reaktionslösung umfasst, wobei die aromatische Verbindung zumindest eine von der aromatischen Säure, dem aromatischen Aldehyd und dem aromatischen Alkohol ist.

14. Verfahren nach Anspruch 13, worin die aromatische Carbonsäure durch Kristallisation abgetrennt wird.

15. Verfahren nach Anspruch 13, worin die aromatische Carbonsäure durch Säurefällung abgetrennt wird.

16. Verfahren nach Anspruch 13, worin das aromatische Aldehyd und der aromatische Alkohol durch Destillation von der Reaktionslösung abgetrennt werden.

17. Verfahren nach Anspruch 13, worin die aromatische Verbindung Xylol ist.

18. Verfahren nach einem der Ansprüche 13 bis 15, worin vor Abtrennung der aromatischen Carbonsäure eine wässrige Phase der Reaktionslösung abgetrennt und entfernt wird.

19. Verfahren nach Anspruch 18, worin die wässrige Phase rückgeführt und im Oxidationsprozess eingesetzt wird.

20. Verfahren nach Anspruch 13, worin - vor Abkühlung der und Kristallausfällung aus der Reaktionslösung - die Lösung mit einer Base versetzt wird, wonach ein gebildetes Salz abgetrennt wird.

21. Verfahren nach Anspruch 20, worin die mit der Base versetzte Reaktionslösung mit Wasser versetzt wird.

22. Verfahren nach Anspruch 16, worin beim Destillieren der Reaktionslösung, aus der die aromatische Carbonsäure entfernt wird, die Destillation nach Entfernen von sauren Komponenten durchgeführt wird.

23. Verfahren nach Anspruch 16, worin der Rückstand, der nach Destillation der Reaktionslösung erhalten wird, zum Einsatz in der Oxidationsreaktion rückgeführt wird.

24. Verfahren nach einem der Ansprüche 16, 22 und 23, das den zusätzlichen Schritt der Destillation des Gemischs, welches das aromatische Aldehyd und den aromatischen Alkohol umfasst und zusätzlich eine aromatische Säure enthält, in Gegenwart einer Base umfasst.

25. Verfahren nach Anspruch 24, worin das aromatische Aldehyd Tolualdehyd ist.

26. Verfahren nach Anspruch 24, worin der aromatische Alkohol Methylbenzylalkohol ist.

27. Verfahren nach einem der Ansprüche 24 bis 26, worin die Base in einer Menge von 10 Gew.-% oder weniger, bezogen auf die Gesamtmenge des aromatischen Aldehyds und des aromatischen Alkohols, zugesetzt wird.

28. Verfahren nach einem der Ansprüche 1 bis 15, das den zusätzlichen Schritt der Reinigung einer rohen aromatischen Carbonsäure, die durch das Verfahren erhalten wird, und der gleichzeitigen Rückführung der Waschlösung zum Einsatz als Rohmaterial umfasst.

29. Verfahren nach Anspruch 28, worin die erhaltene aromatische Carbansäure eine alkylsubstituierte Benzoesäure ist.

30. Verfahren nach Anspruch 29, worin der rohe aromatische Kohlenwasserstoff Xylol und die aromatische Carbonsäure Toluylsäure ist.

31. Verfahren nach Anspruch 30, worin der rohe aromatische Kohlenwasserstoff p-Xylol und die aromatische Carbonsäure p-Toluylsäure ist.

## Revendications

1. Méthode de préparation d'au moins un acide carboxylique aromatique, un aldéhyde aromatique et un alcool aromatique, par oxydation d'au moins un composé aromatique représenté par la formule (I) comme suit : 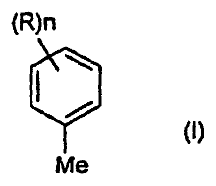 où n est un entier de 1 à 5 et chaque R, indépendamment, est alkyle C₁₋₁₈, aryle C₆₋₁₈, arakyle C₆₋₁₈ ou haloakyle,
la méthode comprenant l'oxydation d'au moins un composé aromatique de formule (I) en tant que matière première avec un gaz contenant de l'oxygène moléculaire en présence d'un catalyseur comprenant un composé d'un métal de transition, une amine tertiaire et un composé de bromure, où le composé d'un métal de transition est présent en une quantité de 0,2% ou moins, calculée en tant qu'une quantité du métal de transition par rapport au poids de la matière première et le rapport pondéral du composé métal de transition : amine tertiaire est dans une gamme de 1:0,1 à 1:100.

2. Méthode selon la revendication 1, où dans la formule (I), chaque R indépendamment est alkyle C₁₋₁₈ ou aralkyle C₆₋₁₈.

3. Méthode selon la revendication 1 ou 2, où la matière première est au moins un composé sélectionné parmi xylène, tolualdéhyde et alcool méthylbenzylique.

4. Méthode selon l'une quelconque des revendications précédentes, où le métal de transition est sélectionné dans le groupe de 5 à 10 dans la Table Périodique à long terme.

5. Méthode selon la revendication 4, où le métal de transition est sélectionné parmi manganèse, tungstène, molybdène, chrome, vanadium, cobalt et cérium.

6. Méthode selon toute revendication précédente, où le composé d'un métal de transition est sélectionné parmi un chlorure, un bromure, un acétate et un sulfate.

7. Méthode selon la revendication 6, où le composé d'un métal de transition est sélectionné parmi le chlorure de cobalt, le bromure de cobalt et l'acétate de cobalt.

8. Méthode selon toute revendication précédente, où le rapport pondéral du composé métal de transition : composé de bromure est dans une gamme de 1:0,1 à 1:100.

9. Méthode selon toute revendication précédente, où de l'eau est ajoutée dans une solution de réaction chargée.

10. Méthode selon la revendication 9, où l'eau est présente en une quantité de 0,01 à 20% en poids par rapport au poids de la matière première.

11. Méthode selon toute revendication précédente, où la réaction d'oxydation est effectuée à une température de 100 à 250°C.

12. Méthode selon toute revendication précédente, où la réaction d'oxydation est effectuée sous une pression de 0,1 à 6 MPa.

13. Méthode selon toute revendication précédente, qui comprend l'étape additionnelle de séparer un composé aromatique de la solution réactionnelle, lequel composé aromatique est au moins un dudit acide aromatique dudit aldéhyde aromatique et dudit alcool aromatique.

14. Méthode selon la revendication 13, où l'acide carboxylique aromatique est séparé par cristallisation.

15. Méthode selon la revendication 13, où l'acide carboxylique aromatique est séparé par précipitation acide.

16. Méthode selon la revendication 16, où l'aldéhyde aromatique et l'alcool aromatique sont séparés de la solution réactionnelle par distillation.

17. Méthode selon la revendication 13, où le composé aromatique est du xylène.

18. Méthode selon l'une quelconque des revendications 13 à 15, où avant la séparation de l'acide carboxylique aromatique, une couche aqueuse de la solution de réaction est séparée et éliminée.

19. Méthode selon la revendication 18, où la couche aqueuse est recyclée et utilisée dans le procédé d'oxydation.

20. Méthode selon la revendication 13, où avant le refroidissement et la précipitation des cristaux de la solution réactionnelle, la solution est mise en contact avec une base avec ensuite séparation du sel formé.

21. Méthode selon la revendication 20, où la solution réactionnelle contactée par la base est encore contactée par de l'eau.

22. Méthode selon la revendication 16, où à la distillation de la solution réactionnelle, d'où est éliminé l'acide carboxylique aromatique, la distillation est effectuée après élimination des composants acides.

23. Méthode selon la revendication 16, où le résidu obtenu après distillation de la solution réactionnelle est recyclé pour une utilisation dans la réaction d'oxydation.

24. Méthode selon l'une quelconque des revendications 16, 22 et 23 qui comprend l'autre étape de distiller le mélange comprenant l'aldéhyde aromatique et l'alcool aromatique contenant additionnellement un acide aromatique en présence d'une base.

25. Méthode selon la revendication 24, où l'aldéhyde aromatique est le tolualdéhyde.

26. Méthode selon la revendication 24, où l'alcool aromatique est l'alcool méthylbenzylique.

27. Méthode selon l'une quelconque des revendications 24 à 26, où la base est ajoutée en une quantité de 10% en poids ou moins par rapport à la quantité totale de l'aldéhyde aromatique et de l'alcool aromatique.

28. Méthode selon l'une quelconque des revendications 1 à 15 qui comprend l'étape additionnelle de purifier un acide carboxylique aromatique brut obtenu par ladite méthode et de recycler simultanément la solution de lavage pour une utilisation comme matière première.

29. Méthode selon la revendication 28, où l'acide carboxylique aromatique obtenu est un acide benzoïque alkyle substitué.

30. Méthode selon la revendication 29, où l'hydrocarbure aromatique brut est le xylène et l'acide carboxylique aromatique est l'acide toluïque.

31. Méthode selon la revendication 30, où l'hydrocarbure aromatique brut est le p-xylène et l'acide carboxylique aromatique est l'acide p-toluïque.
